# EUROPEAN PATENT SPECIFICATION

(11) **EP 3 436 155 B1**
(45) Date of publication and mention of the grant of the patent: **13.10.2021**
(21) Application number: 17708249.2
(22) Date of filing: 02.03.2017
(51) Int. Cl.: A61Q 11/00, A61K 8/19, A61K 8/24, A61K 8/25, A61K 8/26, A61K 8/27

(54) **TOOTHPASTE COMPOSITION**
ZAHNPASTAZUSAMMENSETZUNG
COMPOSITION DE DENTIFRICE

(30) Priority: 31.03.2016 EP 16163164
(43) Date of publication of application: 06.02.2019
(73) Proprietor: Unilever Global IP Limited, Wirral, CH62 AZD (GB); Unilever IP Holdings B.V., 3013 AL Rotterdam (NL)
(72) Inventor: CHANDRASEKARAN, Sembian, Mumbai 400099 (IN); IYER, Meenakshi, Mumbai 400099 (IN); TRIVEDI, Neha, Mumbai 400099 (IN)
(74) Representative: Tansley, Sally Elizabeth
(86) International application number: PCT/EP2017/054856
(87) International publication number: WO 2017/167535

(56) References cited:
- WO-A1-2006/050777
- WO-A2-2013/007571
- FR-A- 1 440 182
- US-A1- 2009 202 453
- US-A1- 2011 229 534
- US-A1- 2014 170 084
- US-A1- 2015 328 094
- US-A1- 2016 000 667

## Description

### Field of the Invention

The present invention relates to a stable toothpaste composition having zinc salt for providing antimicrobial benefits.

### Background of the Invention

Antimicrobial benefit of zinc in personal care compositions is generally recognized. Incorporation of zinc in toothpaste composition provides anti-plaque effects, deriving from antimicrobial properties of the zinc.

Zinc is a relatively non-toxic, non-cumulative and an essential trace element that acts as a plaque inhibitor or as a calculus inhibitory agent when used in toothpaste compositions. Despite the known benefits of zinc salts, their use especially in toothpaste compositions has certain undesirable drawbacks.

The negative effects of incorporating zinc salts in a toothpaste composition is particularly predominant when a calcium based abrasive is present. The calcium based abrasive reacts with the zinc ions resulting in the precipitation of zinc hydroxide and release of carbon dioxide. The gas produced in this reaction inflates the closed container in which the toothpaste composition is stored and will eventually lead to bursting when stored for extended periods of time, for example, between manufacture and use.

Hence, very few attempts have been made in the past to produce a toothpaste composition having a zinc salt and a calcium based abrasive.

WO2013/007571 A2 (Unilever) discloses a toothpaste composition having a selective balance between calcium based abrasive, a copolymer of vinylmethylether and maleic acid and a clay which provides significantly better stability.

GB1319247 A (Beecham Inc, 1973) discloses a non-liquid dental composition which prevents the calcification of plaque into calculus. The dental composition includes a dental vehicle and a zinc, cupric or zirconium complex of a fluorinated β-diketones.

US2015328094 A (Colgate, 2015) discloses a toothpaste composition in which uptake of zinc is increased by incorporating calcium carbonate into the toothpaste based on abrasive silica. It is said to enable enhanced antibacterial, anticavity, enamel erosion protection, breath freshening and other benefits through the composition.

US20160000667 A1 (Colgate, 2016) discloses a toothpaste comprising a first dentifrice comprising a calcium carbonate abrasive and a second dentifrice comprising a zinc ion source in a gel base, wherein the second dentifrice is entrained as a stripe in the first dentifrice.

It is highly desirable to provide a toothpaste composition having antibacterial benefits, which has enhanced acceptance by user. Therefore, it is desirable that the performance of the zinc salt in a toothpaste composition is improved and which delivers maximum benefit without compromising on the other benefits derivable from a toothpaste composition.

It is therefore an object of the present invention to provide a toothpaste composition having calcium based abrasive and a zinc salt as the antimicrobial agent yet which exhibits good storage stability.

An object of the present invention is to provide a toothpaste composition, which effectively deliver antimicrobial benefits of zinc with minimal fluoride ion reduction during storage.

The present inventors have found that a toothpaste composition having calcium based abrasive and a zinc salt effectively delivers the antimicrobial benefits of zinc with minimal stability issues during storage, in the presence of smectite clay and a buffering agent. It is further surprisingly found that in the presence of a smectite clay and a buffering agent the incorporated fluoride ion source is present in a readily available form.

### Summary of the Invention

According to a first aspect of the present invention disclosed is a toothpaste composition having:
a) 10 to 70 wt% calcium based abrasive;
b) a zinc salt; and,
   characterised in that the composition comprises a smectite clay and a buffering agent,
   wherein the composition has a pH from 8.0 to 10.0, and
   wherein the composition comprises a fluoride ion source.

According to a second aspect of the present invention disclosed is a toothpaste composition of the first aspect for use in promoting oral hygiene.

Further disclosed is use of a combination of smectite clay and a buffering agent in a toothpaste composition of the first aspect for effectively delivering antibacterial benefits of zinc salt.

Also disclosed is use of a combination of smectite clay and a buffering agent in a toothpaste composition of the first aspect for effectively delivering zinc salt while providing effective amount of fluoride ions.

### Detailed Description of the Invention

As used herein the term "comprising" encompasses the terms "consisting essentially of" and "consisting of". Where the term "comprising" is used, the listed steps or options need not be exhaustive. Unless otherwise specified, numerical ranges expressed in the format "from x to y" are understood to include x and y. In specifying any range of values or amounts, any particular upper value or amount can be associated with any particular lower value or amount. All percentages and ratios contained herein are calculated by weight unless otherwise indicated. The various features of the present invention referred to in individual sections above apply, as appropriate, to other sections *mutatis mutandis.* Consequently, features specified in one section may be combined with features specified in other sections as appropriate. Any section headings are added for convenience only, and are not intended to limit the disclosure in any way.

According to the first aspect of the present invention disclosed is a toothpaste composition having calcium based abrasive, a zinc salt, a smectite clay, a buffering agent and a fluoride ion source, wherein the composition has a pH from 8.0 to 10.0.

### Toothpaste Composition

Disclosed toothpaste composition preferably has a pH of 8.5 to 10.

Preferably the total water content of the disclosed composition is from 20 wt% to 65 wt% more preferably from 30 wt% to 55 wt%.

Preferably the toothpaste composition has a viscosity in the range of 80,000 to 500,000 cps, when measured at 25°C with a BROOKFIELD® Viscometer using T-bar D-spindle at 5 rpm. Viscosity of more preferred toothpaste compositions is 100,000 to 250,000 cps.

### Calcium Based Abrasive

Disclosed toothpaste composition includes 10 to 70wt% calcium based abrasive. Preferably the calcium based abrasive is more than 20 wt%, more preferably more than 30 wt%, still more preferably more than 35 wt% of the toothpaste composition but preferably less than 65 wt%, more preferably less than 60 wt%, still more preferably less than 55 wt%, further more preferably less than 50 wt% and most preferably less than 40 wt% of the toothpaste compositon.

A preferred calcium based abrasive is fine ground natural chalk (FGNC), which is a form of chalk. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling.

FGNC may be used as the sole calcium based abrasive. However, FGNC may also be used with other calcium based abrasives for some balance of abrasion. Usually the particle size of chalk is from 1 to 60 µm, and preferred sizes range from 1 to 15 µm.

Other preferred calcium containing abrasives include di-calcium phosphate (DCP), calcium pyrophosphate and precipitated calcium carbonate (PCC), which preferably are included at 25 to 55 wt%, more preferably 35 to 50 wt% of the toothpaste composition.

When a combination of calcium based abrasives is desired, it is preferred that FGNC is 35 to 100%, more preferably 75 to 100% and especially from 95 to 100% of the total amount of calcium based abrasives. In such cases, the balance, most preferably, is PCC.

Other abrasives may also be used depending upon the intended degree of abrasion. These include synthetic abrasive polishing agents such as amorphous precipitated silica and silica gels. Other abrasive agents include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

### Zinc salt

Disclosed toothpaste composition includes a zinc salt. Useful zinc salts includes but not limited to zinc chloride, zinc sulphate, zinc nitrate, zinc gluconate, zinc acetate, zinc lactate, zinc oxide, zinc carbonate, zinc phosphate, zinc salicylate, zinc monoglycerolate or a mixture thereof. Mixed salts such as sodium zinc citrate can also be used. Particularly preferred zinc salts include zinc salts of organic acids such as zinc citrate, zinc lactate, zinc maleate, zinc salicylate, zinc gluconate and zinc ascorbate. Preferably, the zinc salt is water soluble or sparingly water soluble, more preferably the zinc salt is water soluble.

It is generally known in the art that the zinc needs to be in soluble form to be efficacious against bacteria and plaque. Most preferred salt is zinc sulphate. Mixtures of different zinc salts can be used.

Preferably the zinc salt is present from 0.1 to 5 wt% of the toothpaste composition, preferably 0.1 to 2 wt% and most preferably the zinc salt is from 0.1 to 0.75 wt% of the toothpaste composition.

### Smectite clay

Disclosed toothpaste composition includes a smectite clay. Smectites constitute a group in the class of natural aluminosilicate minerals known as layered silicates which includes beidellite, bentonite, hectorite, montmorillonite, saponite or stevensite. Preferred smectite clay is selected from phyllosilicates, montmorillonites, bentonites, hectorites and derivatives thereof, purified magnesium aluminium silicates, purified sodium magnesium silicates; organically modified smectites, organically modified montmorillonite clays and mixtures thereof.

Further preferred smectite clay is selected from montmorillonites (bentonites, hectorites and derivatives thereof); purified magnesium aluminium silicates (various grades are commercially available as VEEGUM® from R. T. Vanderbilt Company); purified sodium magnesium silicates (commercially available as LAPONITE® in various grades); organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof. Magnesium aluminium silicate clay are particularly preferred. An example is VEEGUM® HV.

Smectite clays are a class of natural mineral clays which exhibit high swelling/gelling and absorption properties. A macroscopic smectite clay particle is composed of thousands of sandwiched platelets with a central alumina or magnesia layer joined to silica layers. The platelets have negatively charged platelet faces. Lattice discontinuities account for a very slight positive charge on the platelet edges. The net platelet charge is negative and the net negative charge on the platelet is mostly balanced by sodium ions. However, these charge-balancing ions associated with platelet faces are termed "exchangeable" since they can be readily substituted with other cations.

Preferred toothpaste compositions contain 0.2 to 5wt% smectite clay. More preferred composition includes 0.2 to 3 wt% smectite clay and more preferably 0.5 to 1 wt% smectite clay.

### Buffering agent

Disclosed toothpaste composition includes a buffering agent. Preferably, a single buffering agent or a combination of two or more buffering agents is used in the disclosed composition.

Suitable examples of the buffering agent includes but is not limited to alkali metal silicate, alkali metal hydroxide, alkali metal bicarbonate, alkali metal carbonate and alkali metal pyrophosphate, arginine and combinations thereof.

Preferably, the buffering agent is an alkali metal silicate, the alkali metal is sodium or potassium, more preferably sodium. Sodium silicate is generally available as 10% to 40% aqueous solution, most common being a 30% solution. Sodium silicate is available as neutral sodium silicate or alkaline sodium silicate. Preferred toothpaste compositions have neutral sodium silicate. Sodium silicate is available with varying ratios of Na₂O: SiO₂. Sodium silicate with Na₂O: SiO₂ ratio in the range of 2.85 to 3.8 is preferred, still preferably 3.0 to 3.8 and more highly preferred range being 3.25 to 3.5. Preferred toothpaste compositions include 0.1 wt% to 5 wt% silicate (on dry weight basis). Thus, a 30% solution of sodium silicate is added to the composition in an amount in the range of 0.3 to 16 wt%.

It is also preferred that the buffering agent is sodium hydroxide. Preferably the sodium hydroxide is present in an amount of 0.01 to 0.1 wt% more preferably 0.02 to 0.09 wt%, still more preferably 0.04 to 0.08 wt% and further more preferably 0.05 to 0.075 wt% of the total composition. When sodium hydroxide is present in amounts above 0.1 wt% it was found that the viscosity of the composition increased and it was difficult to release the composition from the package.

It is preferred that the buffering agent is a combination of two or more buffering agents selected from but not limited to:
(a) a combination of sodium silicate and tetrasodium pyrophosphate; (b) a combination of sodium hydroxide, sodium bicarbonate and tetrasodium pyrophosphate; (c) a combination of sodium bicarbonate and sodium carbonate; or (d) alkali metal silicate and alkali metal hydroxide preferably a combination of sodium silicate and sodium hydroxide.

Preferably, the buffering agent is 0.25 wt% to 0.75 wt% sodium silicate and 0.25 wt% to 0.75 wt% tetrasodium pyrophosphate; or 0.4 wt% sodium silicate and 0.5 wt% tetrasodium pyrophosphate, based on the total weight of the composition.

Another preferred buffering agent includes a combination of 0.04 wt% to 0.1 wt% sodium hydroxide, 0.25 wt% to 0.75 wt% sodium bicarbonate and 0.25 wt% to 1.5 wt% tetrasodium pyrophosphate based on the total weight of the composition. Also preferred is a combination of 0.04 wt% sodium hydroxide, 0.5 wt% sodium bicarbonate and 0.5 wt% tetrasodium pyrophosphate based on the total weight of the composition, alternatively a combination of 0.06 wt% sodium hydroxide, 0.5 wt% sodium bicarbonate and 0.5 wt% tetrasodium pyrophosphate based on the total weight of the composition.

Yet another preferred buffering agent includes a combination of 0.05 to 0.5 wt% sodium bicarbonate and 0.2 to 0.6 wt% sodium carbonate, also preferred is a combination of 0.1 wt% sodium bicarbonate and 0.4 wt % sodium carbonate, based on the total weight of the composition.

A highly preferred buffering agent includes a combination of 0.1 wt% to 5 wt% sodium silicate and 0.01 wt% to 0.1 wt% sodium hydroxide, also preferred is a combination of 1.5 wt% sodium silicate and 0.075 wt % sodium hydroxide, based on the total weight of the composition.

Preferably the buffering agent is present in an amount of from 0.01 to 30 wt% of the toothpaste composition. Preferably the buffering agent is present in an amount more than 0.07 wt% still preferably more than 1 wt% further preferably more than 1.2 wt% and still more preferably more than 1.5 wt% but preferably less than 10 wt% more preferably less than 8 wt% and further preferably less than 5wt% and most preferably less than 3 wt% of the toothpaste composition.

In order to achieve the desired results, the pH of the disclosed toothpaste compositions is maintained from 8.0 to 10 and preferably from 8.5 to 10. It is believed that when the pH is in these ranges the toothpaste compositions deliver the fluoride ions and zinc ions effectively and these ions do not precipitate out from the solution. Another point of importance of pH is that pH is an indirect indicator of stability of the compositions. Toothpaste compositions which comprise zinc and calcium based abrasive, especially calcium carbonate, are prone to gas generation as a result of unfavourable in situ interaction of zinc with the calcium based abrasive. At or above pH of 8, such interactions are prevented, or at least reduced substantially. Conversely, pH below 8 facilitates such interactions. Further, storage at higher temperature, e.g., 40 °C; often results in drastic or gradual decrease in pH and any such decease is undesirable. It is expected that toothpaste compositions should be able to withstand higher temperature. Therefore storage stability tests are performed during routine R&D and quality control checks. In the case of compositions which pass such tests, it is often observed that there is an upward change in the pH of the compositions. Such a change is acceptable but a decrease below pH of 8 is not acceptable.

Therefore, it is necessary to ensure pH of 8 or above. We have observed that pH of the compositions in accordance with the invention is 8 or above, and the pH remains more or less constant, even after storage, as described earlier.

### Anti-caries agent

The compositions include one or more anti-caries agent. Such agents are typically fluorides. The composition includes a fluoride ion source. It is preferred that the source of fluoride is an alkali-metal salt of monofluorophosphoric acid, preferably sodium monofluorophosphate (SMFP).

SMFP is the fluoride source of choice when it comes to toothpaste compositions having calcium based abrasives, especially chalk; since the alternative, sodium fluoride, reacts with the calcium carbonate to form insoluble calcium fluoride which has limited anti-caries activity. Preferred compositions include 0.01 to 2 wt%, more preferably 0.15 to 1 wt% and especially preferably 0.2 to 0.8 wt% anti-caries agent. It is preferable to maintain the free fluoride ion concentration from 100 to 2000 ppm, preferably from 900 to 1500 ppm. Other preferred anti-caries agents include sodium- and stannous fluoride, aminefluorides, sodium trimetaphosphate and casein.

### Thickening silica

Disclosed composition preferably includes a thickening silica. Preferred thickening silicas include AEROSIL®T series from Degussa or the CAB-O-SIL® series from Cabot Corporation, silica gels such as the SYLODENT® or SYLOX® series from W. R.Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL®, MFIL®-P (From Madhu Silica, India), SIDENT® 22 Sand AEROSIL® 200 (Ex. Evonik Industries), SYLODENT® and PERKASIL® thickening silicas from WR Grace & Company and Tixosil® 43 and 331 from Rhodia, synthetic finely divided pyrogenic silica such as 25 those sold under the trademarks SYLOID® 244, SYLOID® 266 and AEROSIL® D-200.

### Other binders

Preferred toothpaste compositions have a binder, which lends a good structure to the toothpaste. Cellulosic binders are especially preferred. Preferred cellulosic binders include cellulose ethers, which include hydroxyethyl cellulose (HEC), hydroxypropyl cellulose (HPC), ethylhydroxyethyl cellulose (EHEC), carboxymethyl cellulose (CMC), carboxymethylhydroxyethyl cellulose (CMHEC), hydroxypropylhydroxyethyl cellulose (HPHEC), methyl cellulose (MC), methylhydroxypropyl cellulose (MHPC), methylhydroxyethyl cellulose (MHEC), carboxymethylmethyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethylhydroxyethyl cellulose (HMEHEC), hydrophobically modified carboxymethylhydroxyethyl cellulose (HMCMHEC), hydrophobically modified hydroxypropylhydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is sodium carboxymethyl cellulose (SCMC). Particularly preferred sodium carboxymethyl celluloses include those with degree of substitution of from 0.6 to 0.99, preferably from 0.7 to 0.95. Further, preferred SCMCs include those with viscosity of 250 mPa.s to 10000 mPa.s as measured on a Brookfield viscometer TA spindle at 30 rpm, 23 °C and reading after 30 seconds when slurried with flavour in a 1:1 ratio.

In addition to, or as a replacement of cellulosic binder, guar gum or its derivatives could be used, however, such binders are less preferred to the cellulosic ones. Such derivatives include carboxymethyl guar (CM guar), hydroxyethyl guar (HE guar), hydroxypropyl guar (HP guar), carboxymethylhydroxypropyl guar (CMHP guar), cationic guar, hydrophobically modified guar (HM guar), hydrophobically modified carboxymethyl guar (HMCM guar), hydrophobically modified hydroxyethyl guar (HMHE guar), hydrophobically modified hydroxypropyl guar (HMHP guar), cationic hydrophobically modified hydroxypropyl guar (cationic HMHP guar), hydrophobically modified carboxymethylhydroxypropyl guar (HMCMHP guar) and hydrophobically modified cationic guar (HM cationic guar).

Other less preferred gums include xanthan gum, carrageenan and derivatives, such as Irish moss and viscarin, gellan gum, sclerotium gum and derivatives, pullulan, rhamsan gum, welan gum, konjac, curdlan, algin, alginic acid, alginates and derivatives, starch phosphate derivatives, agar and derivatives, gum arabic and derivatives, pectin and derivatives, chitosan and derivatives, karaya gum, locust bean gum, natto gum, tragacanth gum, chitin derivatives, gelatin, betaglucan, dextrin, dextran, cyclodextrin and polyquaterniums, furcellaren gum, ghatti gum, psyllium gum, quince gum, tamarind gum, larch gum, and tara gum.

Binders provide body and binding ability to the compositions. In order to balance the viscosity and the binding ability of the composition, a balance needs to be struck between the smectite clay and the binder, whenever present. For example, if more smectite clay is introduced into the composition, the binder content is preferably lowered. In the case of formulations containing thickening silica and smectite clay and binder, their individual actions need to be taken into consideration because these actions usually produce a cumulative effect. For example, in the case of compositions containing thickening silica, the binder content needs to be carefully controlled otherwise an extremely viscous paste is formed.

In the case of preferred compositions which have cellulosic binder in addition to the smectite clay, the ratio of the amount of smectite clay to the cellulosic binder is in the range of 1:0.2 to 1:0.9. Excessive cellulosic binder leads to compositions which show a tailing effect while dispensing the composition on a toothbrush, which is an undesirable in-use effect. On the other hand, most of the cellulosic binders, and some others like carrageenan are expensive ingredients.

### Humectants

Humectants enhance flavour, prevent harsh taste and provide a fresh and pleasant sensation in the mouth. They also prevent caking. A humectant serves to keep the dentifrice compositions from hardening upon exposure to air and imparts some sweetness to the formulations to further minimize the astringency ascribed to the zinc salt. Preferred levels are from 10 to 70 wt%, more preferably about 25 to 60 wt% by weight of the toothpaste composition. Typical humectants include sorbitol (generally available as 70% aqueous solution), glycerine, maltitol and xylitol. More preferred toothpastes comprises 10 to 30 wt% sorbitol in the form of a 70% aqueous solution. Sorbitol solution supplies sweetness and body to the composition and gives a desirable mouth feel. It may be preferable to use a mixture of glycerine and sorbitol for lubricated mouth feel.

### Antibacterial agent

Preferred compositions may include a further anti-bacterial agent, which may be stannous salts such as stannous pyrophosphate. Further examples of antibacterial agents include quaternary ammonium compounds such as cetylpyridinium chloride; bis-biguanides such as chlorhexidine, chlorhexidine digluconate, hexetidine, octenidine, alexidine, TRICLOSAN® and other halogenated bisphenolic compounds such as 2,2' methylenebis-(4-chloro-6-bromophenol). A particularly preferred antibacterial agent is TRICLOSAN®.

### Preservatives

Toothpastes with calcium based abrasive, especially chalk; are prone to bacterial growth. Certain preservatives, e.g. methyl, ethyl, butyl, propyl and isopropyl esters of parahydroxybenzoic acid may be particularly useful against bacterial growth. A mixture of methyl, ethyl, butyl and propyl esters of parahydroxybenzoic acid is particularly preferred.

The activity of this mixture can be enhanced by adding phenoxyethanol. Formaldehyde and dimethyl hydantoin are other preferred preservatives. Preservatives are generally included at a level of 0.05 to 0.8 wt%.

### Antioxidants

Preferred antioxidants are those which are compatible with other components and are not hazardous to health. These include ascorbic acid, ascorbyl palmitate, thiodipropionic acid, calcium ascorbate, dilauryldithiopropionate, gum guaiac, sodium ascorbate, butylated hydroxyl toluene, butylated hydroxyl anisole, and tocopherols. Mixtures of antioxidants can be used.

When present, the antioxidant is added in a level effective to reduce or mitigate discoloration that would otherwise result from oxidation of the components of the toothpastes. Preferred levels are from 0.01 to 1 wt%.

### Surfactants

Toothpastes generally contain surfactants, also commonly referred to as sudsing agents. Suitable surfactants are those which are reasonably stable and provide foam throughout a wide pH range. The surfactant may be anionic, nonionic, amphoteric, zwitterionic, cationic, or mixtures thereof.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type. Other suitable anionic surfactants are sarcosinates, such as sodium lauroyl sarcosinate, taurates, sodium lauryl sulfoacetate, sodium lauroyl isethionate, sodium laureth carboxylate, and sodium dodecyl benzenesulfonate.

Nonionic surfactants can be broadly defined as compounds produced by the condensation of alkylene oxide groups (hydrophilic in nature) with an organic hydrophobic compound which may be aliphatic or alkyl- aromatic in nature.

Examples of suitable nonionic surfactants include poloxamers (sold under trade name PLURONIC®), polyoxyethylene, polyoxyethylene sorbitan esters (sold under trade name TWEENS®), POLYOXYL® 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials. The nonionic surfactant Poloxamer® 407 is one of the most preferred surfactant because the poloxamer has been discovered to help reduce astringency.

Useful amphoteric surfactants can be broadly described as derivatives of aliphatic secondary and tertiary amines in which the aliphatic radical is a straight chain or branched and wherein one of the aliphatic substituents contains from about 8 to about 18 carbon atoms and one contains an anionic water-solubilizing group, e.g., carboxylate, sulfonate, sulfate, phosphate, or phosphonate. Other suitable amphoteric surfactants are betaines, specifically cocamidopropyl betaine. Mixtures of amphoteric surfactants can also be employed. Preferred levels are from 0.25 to 12 wt%, preferably from 0.5 to 8 wt%, and most preferably from 1 to about 6 wt%.

### Sweetening agents

Toothpastes may also contain a sweetening agent. Preferred sweetening agents include sodium saccharin, aspartame, sucralose, thaumatin, acesulfame potassium, stevioside, stevia extract, paramethoxy cinnamic aldehyde, neohesperidyl dihydrochalcone and perillartine. Typical levels are from 0.005 to 5 wt%, more preferably from 0.01 to 1 wt%.

### Foam modulators

Useful foam modulators include polyethylene glycols (PEGs), also known as polyoxyethylenes. High molecular weight PEGs are suitable, including those having an average molecular weight of 200,000 to 7,000,000 Daltons, for example 500,000 to 5,000,000 Daltons or 1,000,000 to 2,500,000 Daltons. Preferred compositions include 0.1 to 10 wt%, more preferably 0.2 to 5 wt%, and most preferably 0.25 to 2 wt% foam modulator.

### Other common ingredients

Preferred toothpaste composition may include one or more bleaching agents such as peroxy compounds e.g. potassium peroxydiphosphate, effervescing systems such as sodium bicarbonate/citric acid systems, colour change systems, and chelating agents from 0.001 to 6 wt%, preferably at from 0.5 to 4 wt%, which include alkali metal salts of citric acid, alanine, glycine and serine.

The most preferred are the alkali metal salts of citric acid, especially potassium citrate and most preferably tri-potassium citrate.

Liposomes can be used to improve delivery or stability of one or more active ingredients. Preferred compositions may also include one or more of breath strips, sparkles, large silica particles, granules, beads, and flavour encapsulates for enhanced sensory benefits or for visual appeal.

Titanium dioxide can also be added to the composition for opacity. Titanium dioxide is generally included from 0.25 to 5wt%. Coloring agents may also be added. Coloring agent may be in the form of an aqueous solution, preferably 1% coloring agent in a solution of water.

It is preferred that the compositions contain a flavour. Suitable flavoring components include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants are the paramenthane carboxyamide agents such as N-ethyl-p-menthan-3-carboxamide (known commercially as "WS-3®") and mixtures thereof. The flavour content in the composition is generally present from 0.001 to 5wt%.

In accordance with a second aspect is disclosed a toothpaste composition of the first aspect for use in promoting oral hygiene.

Also is disclosed non-therapeutic use of a combination of smectite clay and a buffering agent in a toothpaste composition of the first aspect for effectively delivering antibacterial benefits of zinc salt.

Further is disclosed non-therapeutic use of a combination of smectite clay and a buffering agent in a toothpaste composition of the first aspect for effectively delivering zinc salt while providing effective amount of fluoride ions.

The invention will now be explained with the help of non-limiting exemplary embodiments.

### Examples

### Example 1

Opaque white toothpaste composition having the formulations as described in the Table 1 was prepared by well known method. After preparing, the toothpaste were stored at 40 °C and the stability of the toothpastes was checked. The, pH, viscosity and fluoride content of the comparative compositions (Composition A and B) and the compositions according to the present invention (Composition 1, Composition 2) were measured immediately after preparation and after aonth's storage at 40 °C. All the compositions were subjected to all the tests again after being stored for 2 months and 3 months at 40 °C.

**TABLE 1**

| Ingredients (wt%) | | Toothpaste composition reference code | | | |
|---|---|---|---|---|---|
| | | A | B | 1 | 2 |
| Veegum® HV | | 0 | 0 | 0.8 | 0.8 |
| Zinc sulphate | | 0.75 | 0.75 | 0.75 | 0.75 |
| Potassium nitrate | | 0.5 | 0.5 | 0.5 | 0.5 |
| Sodium hydroxide (buffering agent) | | 0 | 0 | 0 | 0.075 |
| Sorbitol (70% solution) | | 15 | 15 | 15 | 15 |
| Thickening silica (MFIL®) | | 4.5 | 4.5 | 2 | 2 |
| Sodium carboxy methyl cellulose | | 0.625 | 0.625 | 0.4 | 0.4 |
| Fine ground natural chalk | | 40 | 40 | 40 | 40 |
| Sodium lauryl sulphate | | 2.5 | 2.5 | 2.5 | 2.5 |
| sodium monofluorophosphate | | 0.76 | 0.76 | 0.76 | 0.76 |
| Sodium silicate (buffering agent) | | 0 | 0.45 | 0.45 | 0.45 |
| Flavour | | 1.1 | 1.1 | 1.1 | 1.1 |
| Water | | 33.52 | 33.52 | 33.94 | 33.87 |
| Other minors to* | | 100 | 100 | 100 | 100 |

| | Immediately after preparation | | | | |
|---|---|---|---|---|---|
| zinc content (ppm) | | 1700 | 1700 | 1700 | 1700 |
| Flouride content (ppm) | | 1000 | 1000 | 1000 | 1000 |
| Viscosity (in cps at 25 °C) | | 100,000 | 174,000 | 174,000 | 174,000 |
| pH of the toothpaste | | 7.21 | 7.90 | 8.20 | 8.64 |

| | After 1 month storage at 40 °C | | | | |
|---|---|---|---|---|---|
| zinc content (ppm) | | 1707 | 1710 | 1790 | 1721 |
| Flouride content (ppm) | | 437 | 780 | 795 | 901 |
| Viscosity (in cps at 25 °C) | | 163,000 | 192,000 | 362,000 | 272,000 |
| pH of the toothpaste | | 7.11 | 7.83 | 8.07 | 8.36 |
| pH of the toothpaste after 2 months' storage at 40 °C | | 7.27 | 7.96 | 8.16 | 8.29 |
| pH of the toothpaste after 3 months' storage at 40 °C | | 6.90 | 8.03 | 8.09 | 8.27 |

| | | | | | |
|---|---|---|---|---|---|
| *this includes preservative (benzyl alcohol) and sweetening agent (sodium saccharin) and other minors. ** Compositions A and B are outside the scope of the present invention ** Compositions 1 and 2 are inside the scope of the invention | | | | | |

The data in Table 1 shows that the comparative composition A which has no buffering agent and no smectite clay has significantly reduced levels of fluoride after one month of storage while the compositions according to the present invention having a smectite clay and a buffering agent (composition 1 having sodium silicate as the buffering agent and composition 2 having sodium silicate and sodium hydroxide as the buffering agent) shows significantly higher levels of fluoride content after storage. In addition, the data in Table 1 further indicates how each composition responds to the storage stability tests. The pH of compositions A as well as B is less than 8 *ab initio.* The pH is seen to drop further upon storage. On the other hand, pH of compositions 1 as well as 2 is more than 8 and upon storage, the respective pH tends to increase which is a beneficial effect as far as overall stability of the compositions is concerned.

## Claims

1. A toothpaste composition comprising:
a) 10 to 70wt% calcium based abrasive; and,
b) a zinc salt;
**characterised in that** the composition comprises a smectite clay and a buffering agent
wherein the composition has a pH from 8.0 to 10.0; and
wherein the composition comprises a fluoride ion source.

2. A composition as claimed in claim 1 wherein the zinc salt is a water soluble zinc salt.

3. A composition as claimed in claim 2 wherein the zinc salt is selected from zinc chloride, zinc sulphate, zinc nitrate, zinc citrate, zinc gluconate, zinc acetate, zinc lactate, zinc salicylate, zinc gluconate, zinc ascorbate or mixtures thereof.

4. A composition as claimed in any one of the preceding claims wherein the smectite clay is selected from phyllosilicates, montmorillonites, bentonites, hectorites, purified sodium magnesium silicates, purified aluminium magnesium silicates, organically modified smectites, organically modified montmorillonite clays and mixtures thereof.

5. A composition as claimed in claim 4 wherein the smectite clay is magnesium aluminium silicate clay.

6. A composition as claimed in any one of the preceding claims wherein the buffering agent is selected from alkali metal silicate, alkali metal hydroxide, alkali metal bicarbonate, alkali metal carbonate and alkali metal pyrophosphate, arginine and combinations thereof.

7. A composition as claimed in claim 6 wherein the buffering agent is selected from (a) combination of sodium silicate and tetrasodium pyrophosphate; (b) a combination of sodium hydroxide, sodium bicarbonate and tetrasodium pyrophosphate; (c) a combination of sodium bicarbonate and sodium carbonate; or (d) alkali metal silicate and alkali metal hydroxide preferably a combination of sodium silicate and sodium hydroxide.

8. A composition as claimed in claim 7 wherein the buffering agent is a combination of 0.01 to 0.1 wt% sodium hydroxide and 0.1 to 5 wt% sodium silicate by weight of the composition.

9. A composition as claimed in any one of the preceding claims comprising 0.1 to 5 wt% zinc salt.

10. A composition as claimed in any one of the preceding claims comprising 0.2 to 5 wt% smectite clay.

11. A toothpaste composition for use in promoting oral hygiene, comprising:
a) 10 to 70wt% calcium based abrasive; and,
b) a zinc salt;
**characterised in that** the composition comprises a smectite clay and a buffering agent
wherein the composition has a pH from 8.0 to 10.0; and wherein the composition comprises a fluoride ion source.

## Patentansprüche

1. Zahnpastazusammensetzung, umfassend:
a) 10 bis 70 Gew.-% Scheuermittel auf Calciumbasis; und
b) ein Zinksalz;
**dadurch gekennzeichnet, dass** die Zusammensetzung einen Smektit-Ton und ein Puffermittel umfasst,
wobei die Zusammensetzung einen pH-Wert von 8,0 bis 10,0 aufweist, und
wobei die Zusammensetzung eine Fluoridionenquelle umfasst.

2. Zusammensetzung wie in Anspruch 1 beansprucht, wobei das Zinksalz ein wasserlösliches Zinksalz ist.

3. Zusammensetzung wie in Anspruch 2 beansprucht, wobei das Zinksalz ausgewählt ist aus Zinkchlorid, Zinksulfat, Zinknitrat, Zinkcitrat, Zinkgluconat, Zinkacetat, Zinklactat, Zinksalicylat, Zinkgluconat, Zinkascorbat oder Mischungen davon.

4. Zusammensetzung wie in irgendeinem der vorhergehenden Ansprüche beansprucht, wobei der Smektit-Ton ausgewählt ist aus Phyllosilicaten, Montmorilloniten, Bentoniten, Hectoriten, gereinigten Natriummagnesiumsilicaten, gereinigten Aluminiummagnesiumsilicaten, organisch modifizierten Smektiten, organisch modifizierten Montmorillonit-Tonen und Mischungen davon.

5. Zusammensetzung wie in Anspruch 4 beansprucht, wobei der Smektit-Ton Magnesiumaluminiumsilicatton ist.

6. Zusammensetzung wie in irgendeinem der vorhergehenden Ansprüche beansprucht, wobei das Puffermittel ausgewählt ist aus Alkalimetallsilicat, Alkalimetallhydroxid, Alkalimetallbicarbonat, Alkalimetallcarbonat und Alkalimetallpyrophosphat, Arginin und Kombinationen davon.

7. Zusammensetzung wie in Anspruch 6 beansprucht, wobei das Puffermittel ausgewählt ist aus (a) einer Kombination von Natriumsilicat und Tetranatriumpyrophosphat; (b) einer Kombination von Natriumhydroxid, Natriumbicarbonat und Tetranatriumpyrophosphat; (c) einer Kombination von Natriumbicarbonat und Natriumcarbonat; oder (d) Alkalimetallsilicat und Alkalimetallhydroxid, bevorzugt einer Kombination aus Natriumsilicat und Natriumhydroxid.

8. Zusammensetzung wie in Anspruch 7 beansprucht, wobei das Puffermittel eine Kombination von 0,01 bis 0,1 Gew.-% Natriumhydroxid und 0,1 bis 5 Gew.-% Natriumsilicat, bezogen auf das Gewicht der Zusammensetzung, ist.

9. Zusammensetzung wie in irgendeinem der vorhergehenden Ansprüche beansprucht, umfassend 0,1 bis 5 Gew.-% Zinksalz.

10. Zusammensetzung wie in irgendeinem der vorhergehenden Ansprüche beansprucht, umfassend 0,2 bis 5 Gew.-% Smektit-Ton.

11. Zahnpastazusammensetzung zur Verwendung zur Förderung der Mundhygiene, umfassend:
a) 10 bis 70 Gew.-% Scheuermittel auf Calciumbasis; und
b) ein Zinksalz;
**dadurch gekennzeichnet, dass** die Zusammensetzung einen Smektit-Ton und ein Puffermittel umfasst,
wobei die Zusammensetzung einen pH-Wert von 8,0 bis 10,0 aufweist, und
wobei die Zusammensetzung eine Fluoridionenquelle umfasst.

## Revendications

1. Composition de pâte dentifrice comprenant :
a) 10 à 70 % en masse d'abrasif à base de calcium ; et,
b) un sel de zinc ;
**caractérisée en ce que** la composition comprend une argile de smectite et un agent tampon
dans laquelle la composition présente un pH de 8,0 à 10,0 ; et
dans laquelle la composition comprend une source d'ion fluorure.

2. Composition selon la revendication 1, dans laquelle le sel de zinc est un sel de zinc soluble dans l'eau.

3. Composition selon la revendication 2, dans laquelle le sel de zinc est choisi parmi du chlorure de zinc, sulfate de zinc, nitrate de zinc, citrate de zinc, gluconate de zinc, acétate de zinc, lactate de zinc, salicylate de zinc, gluconate de zinc, ascorbate de zinc ou mélanges de ceux-ci.

4. Composition selon l'une quelconque des revendications précédentes, dans laquelle l'argile de smectite est choisie parmi des phyllosilicates, montmorillonites, bentonites, hectorites, silicates de sodium magnésium purifiés, silicates d'aluminium magnésium purifiés, smectites organiquement modifiées, argiles de montmorillonite organiquement modifiées et mélanges de ceux-ci.

5. Composition selon la revendication 4, dans laquelle l'argile de smectite est une argile de silicate de magnésium aluminium.

6. Composition selon l'une quelconque des revendications précédentes, dans laquelle l'agent tampon est choisi parmi un silicate de métal alcalin, hydroxyde de métal alcalin, bicarbonate de métal alcalin, carbonate de métal alcalin et pyrophosphate de métal alcalin, arginine et combinaisons de ceux-ci.

7. Composition selon la revendication 6, dans laquelle l'agent tampon est choisi parmi (a) combinaison de silicate de sodium et pyrophosphate de tétrasodium ; (b) une combinaison d'hydroxyde de sodium, bicarbonate de sodium et pyrophosphate de tétrasodium ; (c) une combinaison de bicarbonate de sodium et carbonate de sodium ; ou (d) silicate de métal alcalin et hydroxyde de métal alcalin de préférence une combinaison de silicate de sodium et hydroxyde de sodium.

8. Composition selon la revendication 7, dans laquelle l'agent tampon est une combinaison de 0,01 à 0,1 % en masse d'hydroxyde de sodium et de 0,1 à 5 % en masse de silicate de sodium en masse de la composition.

9. Composition selon l'une quelconque des revendications précédentes comprenant de 0,1 à 5 % en masse de sel de zinc.

10. Composition selon l'une quelconque des revendications précédentes comprenant de 0,2 à 5 % en masse d'argile de smectite.

11. Composition de pâte dentifrice pour une utilisation dans la promotion de l'hygiène orale, comprenant :
a) de 10 à 70 % en masse d'abrasif à base de calcium ; et,
b) un sel de zinc ;
**caractérisée en ce que** la composition comprend une argile de smectite et un agent tampon
dans laquelle la composition présente un pH de 8,0 à 10,0 ; et dans laquelle la composition comprend une source d'ion fluorure.
